# EUROPEAN PATENT SPECIFICATION

(11) **EP 0 638 649 B1**
(45) Date of publication and mention of the grant of the patent: **17.11.1999**
(21) Application number: 94202285.6
(22) Date of filing: 05.08.1994
(51) Int. Cl.: C12P 35/02, C12P 37/06, C12R 1/05

(54) **New use of alcaligenes faecalis penicillin G acylase**
Neue Anwendung von Alcaligenes faecalis Penicillin G Acylase
Nouvelle application de penicillin G acylase d'alcaligènes faecalis

(30) Priority: 05.08.1993 EP 93202318
(43) Date of publication of application: 15.02.1995
(73) Proprietor: DSM N.V., 6411 TE Heerlen (NL)
(72) Inventor: De Vroom, Erik, NL-2312 KZ Leiden (NL); van der Mey, Margaretha, NL-2231 ND Rijnsburg (NL)
(74) Representative: Visser-Luirink, Gesina, Dr.

(56) References cited:
- EP-A- 0 453 047
- EP-A- 0 453 048
- FR-A- 1 564 647
- GB-A- 957 449
- GB-A- 957 685
- US-A- 3 239 427
- US-A- 3 272 715
- J. Antibiotics, Vol. 31(8), 1978, pp. 783-788

## Description

### Technical field

The present invention relates to the use of Alcaligenes faecalis penicillin G acylase for the hydrolysis of the optionally substituted phenyl- or heterocyclic acetyl side chain of some penicillanic and cephalosporanic acids and derivatives thereof.

### Background and relevant literature

Penicillin G acylase (benzylpenicillin amidohydrolase, also named penicillin amidase; EC 3.5.1.11) is an enzyme used commercially to hydrolyse penicillin G or 3'-desacetoxycephalosporin G to phenylacetic acid and 6-aminopenicillanic acid (6-APA) or 7-aminodesacetoxycephalosporanic acid (7-ADCA), respectively, the most important intermediates for the industrial production of semi-synthetic penicillins and cephalosporins. This enzyme also catalyses the reverse reaction, viz. the N-acylation of 6-APA and 7-ADCA with organic esters to generate their corresponding N-acetylated penicillin and 3-cephem compounds, respectively. See the reviews of Vandamme, E.J., In: Microbial Enzymes and Bioconversions, E.H. Rose (Ed.), Economic Microbiology 5, 467-552 (1980); and P.B. Mahajan, Appl. Biochem. Biotechnol. 1, 83-86 (1982).

In NL-7502291, DE-2458746, NL-7414302, NL-7416549, NL-7117696 and DE-3316796 deacylation of cephalosporins with substituents on the 3'-position, viz. a substituted -CH₂ group, has been described. However, the yields mentioned are not higher than 92%.

Deacylation activity of E. coli penicillin acylase, applied on an oxidized form of benzylpenicillin, has been described in Tetrah. Lett. 29(36), 4623-4624 (1988). However, a special protection of the carboxy group has been taught. In J. Antibiotics 31(8), 783-788 (1978) it was mentioned that the (S)sulphoxides and the sulphone of benzylpenicillin were not hydrolysed by the E. coli enzyme (penicillin G acylase) preparation. G. Alvaro et al. (Enzyme Microb. Technol. 13, 210-214 (1991) disclose the application of said inertia of penicillin G sulphoxide towards both Escherichia coli and Kluyveromyces citrophila penicillin G acylase by using the same as stabilizing agent upon the immobilization of penicillin G acylase.

In EP-A-0453047 a new penicillin G acylase originating from Alcaligenes faecalis has been disclosed.

Besides the good stability and high specific activity of said Alcaligenes faecalis penicillin G acylase, the enzymatic activity of the same on carboxyl unprotected penicillins with optionally substituted G side chains on the 6-position, and cephalosporins with optionally substituted G side chains on the 7-position and optionally substituted 3-methyl side chains has never been taught or suggested.

Furthermore, in spite of the article in J. Antibiotics cited above, it has been found that, surprisingly, the Alcaligenes faecalis penicillin G acylase enzyme can be applied to deacylate S-oxidized penam and cephem compounds as well as sulphones.

### Summary of the invention

The present invention provides the use of Alcaligenes faecalis penicillin G acylase for the deacylation of the 6- or 7-side chain of, respectively, the penam or cephem compounds of formula (I): 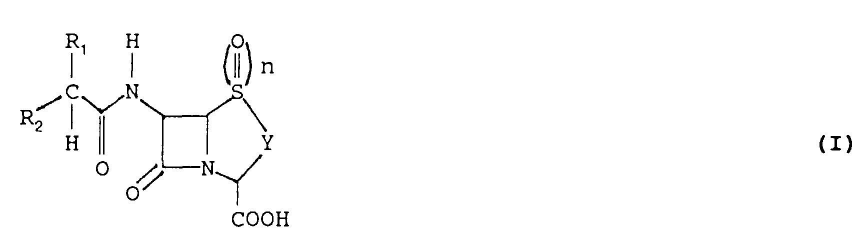 with
R₁ is hydrogen, hydroxy, amine, halogen or lower alkyl;
R₂ is an optionally substituted phenyl or 5- or 6-membered heterocyclic ring;
Y is 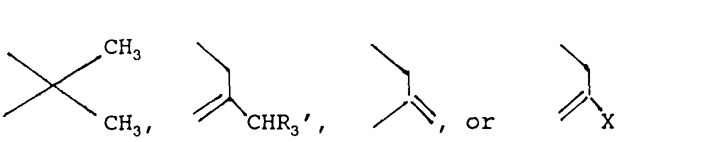 with
   R₃' is (lower)alkylidene; and
   X is hydrogen, halogen or (lower)alkoxy or optionally substituted methyl;
n is 1 or 2.

### Specific embodiments

The Alcaligenes faecalis penicillin G acylase strain has been isolated as described in EP-A-0453047.

The acylase activity is independent of the substituents at the 3-position of the cephem compounds. These substituents are, for instance, hydrogen, halogen, (lower)alkoxy, methyl or methyl substituted with, for instance, (lower)alkoxy, (lower)alkylthio, (lower)alkanoyloxy, (lower)alkanoylthio, halogen or S-R₄ where R₄ is an optionally substituted heterocyclic ring.

By lower throughout this application is meant 1-6 carbon atoms.

The deacylation reaction is to be carried out on penam and cephem compounds, having amino side chains on the 6- and 7-positions, respectively, substituted with an optionally substituted phenyl or 5- or 6-membered heterocyclic ring. The substituents are, for instance, halogen, (lower)alkoxy, methyl or amine.

A heterocyclic ring is defined throughout the application as an unsaturated ring structure comprising at least one nitrogen, sulphur or oxygen atom, as for instance 2-thienyl.

The deacylation reaction can be carried out at 20-50°C, more preferably at 25-40°C, at a pH of 5-9, preferably about 7. The pH of the reaction mixture is brought at this value by the addition of a basic solution, preferably comprising an (earth)alkali metal hydroxide or ammonia.

In some cases it is preferable to extract the product solutions obtained with an inert organic solvent, preferably 1-butanol, ethylacetate, butylacetate, methylisobutyl ketone or toluene at a pH of 0.1-2. The reaction mixture is acidified by using a concentrated solution of inorganic acid, for instance, hydrogen chloride, hydrogen bromide or sulphur acid. Then the organic phase is back-extracted with water. The pH of the combined aqueous layers is brought to about 3 with a concentrated basic solution, resulting in the formation of a slurry containing white crystals. Said slurry is stirred for about half an hour to two hours, at 0-5°C, preferably at 0-2°C. After filtering, washing and drying the crystals are isolated, in a yield of > ± 75%, but in most cases > 90%. The hydrolysed compounds are obtained in high yields with high purities.

Preferably, in industrial processes penicillin G acylase is used in immobilized form. The carrier on which it is immobolized typically comprises gelatin optionally with chitosan, aluminum oxide, silicium oxide, ion exchange resins or acrylate beads, such as, for instance, EUPERGIT^{®}.

The following examples are only given by way of illustration.

### Example 1

### Enzymatic hydrolysis of the phenylacetyl side chain of 6β-phenylacetamidopenicillanic acid-1,1-dioxide

A suspension of 46.0 g (126 mmoles) of 6β-phenylacetamidopenicillanic acid-1,1-dioxide in 200 ml water is brought to 32°C and the pH is adjusted to 7.5 by the addition of a 1M solution of NaOH in water. Immobilized Alcaligenes faecalis penicillin G acylase (63 g, weighed after filtering and washing the beads with 630 ml water) is added and the mixture is stirred at 32°C for 5 h while maintaining the pH of the solution at 7.5 with 174 ml of a 1M solution of NaOH in water. Immobilized Alcaligenes faecalis penicillin G acylase is removed by filtration. HPLC assay of the thus obtained clear red solution indicated the presence of 16.4 g (66.1 mmoles, 52.5%) of 6β-aminopenicillanic acid-1,1-dioxide and 13.5 g (99.2 mmoles, 78.7%) of phenylacetic acid.

### Example 2

### Enzymatic hydrolysis of the phenylacetyl side chain of 6β-phenylacetamidopenicillanic acid-1β-oxide

A suspension of 774 mg (2.21 mmoles) of 6β-phenylacetamidopenicillanic acid-1β-oxide in 22 ml water is brought to 32°C and the pH is adjusted to 7.5 by the addition of a 0.1M solution of NaOH in water. Immobilized Alcaligenes faecalis penicillin G acylase (2.2 g, weighed after filtering and washing the beads with 44 ml water) is added and the mixture is stirred at 32°C for 4 h while maintaining the pH of the solution at 7.5 with 22.3 ml of a 0.1M solution of NaOH in water. Immobilized Alcaligenes faecalis penicillin G acylase is removed by filtration. HPLC assay of the thus obtained clear solution indicated the presence of 300 mg (2.20 mmoles, 99.6%) of phenylacetic acid.

### Example 3

### Enzymatic hydrolysis of the phenylacetyl side chain of 7β-phenylacetamidodesacetoxycephalosporanic acid-1β-oxide

A suspension of 704 mg (2.02 mmoles) of 7β-phenylacetamidodesacetoxycephalosporanic acid-1β-oxide in 20 ml water is brought to 32°C and the pH is adjusted to 7.5 by the addition of a 0.1M solution of NaOH in water. Immobilized Alcaligenes faecalis penicillin G acylase (2.0 g, weighed after filtering and washing the beads with 40 ml water) is added and the mixture is stirred at 32°C for 3 h while maintaining the pH of the solution at 7.5 with 22.1 ml of a 0.1M solution of NaOH in water. Immobilized Alcaligenes faecalis penicillin G acylase is removed by filtration. HPLC assay of the thus obtained clear solution indicated the presence of 274 mg (2.01 mmoles, 99.5%) of phenylacetic acid.

### Example 4

### Enzymatic hydrolysis of the phenylacetyl side chain of 7β-phenylacetamidodesacetoxycephalosporanic acid-1α-oxide

A suspension of 696 mg (2.00 mmoles) of 7β-phenylacetamidodesacetoxycephalosporanic acid-1α-oxide in 20 ml water is brought to 32°C and the pH is adjusted to 7.5 by the addition of a 0.1M solution of NaOH in water. Immobilized Alcaligenes faecalis penicillin G acylase (2.0 g, weighed after filtering and washing the beads with 40 ml water) is added and the mixture is stirred at 32°C for 10 h while maintaining the pH of the solution at 7.5 with 15.1 ml of a 0.1M solution of NaOH in water. Immobilized Alcaligenes faecalis penicillin G acylase is removed by filtration. HPLC assay of the thus obtained slightly troubled solution indicated the presence of 268 mg (1.97 mmoles, 98.5%) of phenylacetic acid.

### Example 5

### Enzymatic hydrolysis of the phenylacetyl side chain of 7β-phenylacetamido-3'-methylenedesacetoxycephalosporanic acid

A suspension of 13.8 g (40.1 mmoles) of 7β-phenylacetamido-3'-methylenedesacetoxycephalosporanic acid in 400 ml water is brought to 32°C and the pH is adjusted to 7.5 by the addition of a 1M solution of NaOH in water. Immobilized Alcaligenes faecalis penicillin G acylase (20 g, weighed after filtering and washing the beads with 200 ml water) is added and the mixture is stirred at 32°C for 3 h while maintaining the pH of the solution at 7.5 with 39.8 ml of a 1M solution of NaOH in water. Immobilized Alcaligenes faecalis penicillin G acylase is removed by filtration. HPLC assay of the thus obtained clear solution indicated the presence of 8.76 g (38.7 mmoles, 96.4%) of 7β-amino-3'-methylenedesacetoxycephalosporanic acid and 5.41 g (39.7 mmoles, 99.0%) of phenylacetic acid. Water is added to the aqueous solution to obtain a final volume of 800 ml and this solution is extracted with 160 ml cold (5°C) 1-butanol. After separation of the organic phase, the aqueous phase is added at 5°C to 160 ml water-saturated 1-butanol. During the above procedure the pH of the resulting two phase system is kept at 0.4 by the addition of a 6M solution of HCl in water. After separation of the organic phase, the aqueous phase is washed with 2 x 80 ml water-saturated 1-butanol. The combined organic layers are back-extracted with 80 ml water. At 10°C, the combined aqueous phases are slowly added to 40 ml water while maintaining the pH of the resulting solution at 3.0 with a 4M solution of NaOH in water. During this procedure, white crystals are formed. The thus obtained slurry is stirred for 1 h at 0-2°C and the product is collected by filtration and washed with 80 ml water and 80 ml acetone. After drying for 24 h at 30°C under vacuum, 9.22 g of product is obtained. ¹H NMR assay indicated the content of the product to be 93%. The yield is therefore 94.5%.

### Example 6

### Enzymatic hydrolysis of the phenylacetyl side chain of 7β-phenylacetamido-3'-ethylidenedesacetoxycephalosporanic acid

A suspension of 7.17 g (20.0 mmoles) of 7β-phenylacetamido-3'-ethylidenedesacetoxycephalosporanic acid in 200 ml water is brought to 32°C and the pH is adjusted to 7.5 by the addition of a 1M solution of NaOH in water. Immobilized Alcaligenes faecalis penicillin G acylase (10 g, weighed after filtering and washing the beads with 100 ml water) is added and the mixture is stirred at 32°C for 3 h while maintaining the pH of the solution at 7.5 with 18.8 ml of a 1M solution of NaOH in water. Immobilized Alcaligenes faecalis penicillin G acylase is removed by filtration. HPLC assay of the thus obtained clear solution indicated the presence of 4.33 g (18.0 mmoles, 90.0%) of 7β-amino-3'-ethylidenedesacetoxycephalosporanic acid and 2.51 g (18.4 mmoles, 92.0%) of phenylacetic acid. The aqueous solution is extracted with 40 ml cold (5°C) 1-butanol. After separation of the organic phase, the aqueous phase is added at 5°C to 40 ml water-saturated 1-butanol. During the above procedure, the pH of the resulting two phase system is kept at 0.8 by the addition of a 6M solution of HCl in water. After separation of the organic phase, the aqueous phase is washed with 20 ml water-saturated 1-butanol. At 10°C, the aqueous phase is slowly added to 10 ml water while maintaining the pH of the resulting solution at 3.0 with a 4M solution of NaOH in water. During this procedure, white crystals are formed. The thus obtained slurry is stirred for 1 h at 0-2°C and the product is collected by filtration and washed with 20 ml water and 20 ml acetone. After drying for 24 h at 30°C under vacuum, 4.47 g of product is obtained. ¹H NMR assay indicated the content of the product to be 91.2%. The yield is therefore 84.8%.

### Example 7

### Enzymatic hydrolysis of the phenylacetyl side chain of 7β-phenylacetamido-3'-nordesacetoxycephalosporanic acid

A suspension of 637 mg (2.00 mmoles) of 7β-phenylacetamido-3'-nordesacetoxycephalosporanic acid in 20 ml water is brought to 32°C and the pH is adjusted to 7.5 by the addition of a 0.5M solution of NaOH in water. Immobilized Alcaligenes faecalis penicillin G acylase (2.0 g, weighed after filtering and washing the beads with 100 ml water) is added and the mixture is stirred at 32°C for 1.5 h while maintaining the pH of the solution at 7.5 with 19.6 ml of a 0.1M solution of NaOH in water. 1 g of immobilized Alcaligenes faecalis penicillin G acylase is added and stirring is continued for 2 h. The total amount of 0.1M NaOH consumed is 21.1 ml. Immobilized Alcaligenes faecalis penicillin G acylase is removed by filtration and washed with 2 x 1 ml water. The combined filtrate is washed with 5 ml of 1-butanol and the 1-butanol layer is extracted with 2 x 1.5 ml water. The combined aqueous layers are added at 20°C to 7.5 ml water-saturated 1-butanol. During the above procedure, the pH of the resulting two phase system is kept at 1.0 by the addition of a 1M solution of HCl in water. After separation of the phases, the 1-butanol phase is extracted with 2 x 1.2 ml of water at pH 1.0. The pH of the combined aqueous phases is raised to 3.0 with a 4M solution of NaOH in water. The solution is concentrated by repeated evaporation with 1-butanol and the residue is dried under reduced pressure and suspended in acetone. The undissolved material was obtained by filtration to give, after drying, 535 mg of material which was triturated at 0°C with a mixture of acetone/water (65/35, v/v) and suspended in methanol. The desired compound (85 mg) was obtained by filtration and washed with ether. Infrared and ¹H NMR spectroscopy indicated that the compound was identical with an authentic sample prepared through chemical deacylation.

### Example 8

### Enzymatic hydrolysis of the phenylacetyl side chain of 7β-phenylacetamido-3-methylenecepham-4β-carboxylic acid

A suspension of 1.01 g (2.59 mmoles) of 7β-phenylacetamido-3-methylenecepham-4β-carboxylic acid in 26 ml water is brought to 32°C and the pH is adjusted to 7.5 by the addition of a 0.1M solution of NaOH in water. Immobilized Alcaligenes faecalis penicillin G acylase (2.6 g, weighed after filtering and washing the beads with 50 ml water) is added and the mixture is stirred at 32°C for 3 h while maintaining the pH of the solution at 7.5 with 28.1 ml of a 0.1M solution of NaOH in water. Immobilized Alcaligenes faecalis penicillin G acylase is removed by filtration. HPLC assay of the thus obtained clear solution indicated the presence of 525 mg (2.45 mmoles, 94.6%) of 7β-amino-3-methylenecepham-4β-carboxylic acid and 335 mg (2.46 mmoles, 95.0%) of phenylacetic acid.

### Example 9

### Enzymatic hydrolysis of the D-α-aminophenylacetyl side chain of 6β-(D-α-aminophenylacetamido)penicillanic acid (ampicillin)

A suspension of 698 mg (2.00 mmoles) of 6β-(D-α-aminophenylacetamido)penicillanic acid in 20 ml water is brought to 32°C and the pH is adjusted to 7.5 by the addition of a 0.1M solution of NaOH in water. Immobilized Alcaligenes faecalis penicillin G acylase (2.0 g, weighed after filtering and washing the beads with 40 ml water) is added and the mixture is stirred at 32°C for 20 min while maintaining the pH of the solution at 7.5 with 6.90 ml of a 0.1M solution of NaOH in water. Immobilized Alcaligenes faecalis penicillin G acylase is removed by filtration. HPLC assay of the thus obtained clear solution indicated the presence of 320 mg (1.48 mmoles, 74.0%) of 6β-aminopenicillanic acid and 248 mg (1.64 mmoles, 82.0%) of phenylglycine.

### Example 10

### Enzymatic hydrolysis of the D-α-aminophenylacetyl side chain of 7β-(D-α-aminophenylacetamido)desacetoxycephalosporanic acid (cephalexin)

A suspension of 700 mg (2.02 mmoles) of 7β-(D-α-aminophenylacetamido)desacetoxycephalosporanic acid in 20 ml water is brought to 32°C and the pH is adjusted to 7.5 by the addition of a 0.1M solution of NaOH in water. Immobilized Alcaligenes faecalis penicillin G acylase (2.0 g, weighed after filtering and washing the beads with 40 ml water) is added and the mixture is stirred at 32°C for 20 min while maintaining the pH of the solution at 7.5 with 6.16 ml of a 0.1M solution of NaOH in water. Immobilized Alcaligenes faecalis penicillin G acylase is removed by filtration. HPLC assay of the thus obtained clear solution indicated the presence of 401 mg (1.87 mmoles, 92.6%) of 7β-aminocephalosporanic acid and 295 mg (1.95 mmoles, 96.5%) of phenylglycine.

### Example 11

### Enzymatic hydrolysis of the D-α-amino-p-hydroxyphenylacetyl side chain of 6β-(D-α-amino-p-hydroxyphenylacetamido)penicillanic acid (amoxycillin)

A suspension of 731 mg (2.00 mmoles) of 6β-(D-α-amino-p-hydroxyphenylacetamido)penicillanic acid in 20 ml water is brought to 32°C and the pH is adjusted to 7.5 by the addition of a 0.1M solution of NaOH in water. Immobilized Alcaligenes faecalis penicillin G acylase (2.0 g, weighed after filtering and washing the beads with 40 ml water) is added and the mixture is stirred at 32°C for 4 h while maintaining the pH of the solution at 7.5 with 15.5 ml of a 0.1M solution of NaOH in water. Immobilized Alcaligenes faecalis penicillin G acylase is removed by filtration. HPLC assay of the thus obtained clear solution indicated the presence of 407 mg (1.88 mmoles, 94.0%) of 6β-aminopenicillanic acid.

### Example 12

### Enzymatic hydrolysis of the α-bromophenylacetyl side chain of 7β-(α-bromophenylacetamido)desacetoxycephalosporanic acid-1β-oxide

A suspension of 855 mg (2.00 mmoles) of 7β-(α-bromophenylacetamido)desacetoxycephalosporanic acid-1β-oxide in 20 ml water is brought to 32°C and the pH is adjusted to 7.5 by the addition of a 0.1M solution of NaOH in water. Immobilized Alcaligenes faecalis penicillin G acylase (2.0 g, weighed after filtering and washing the beads in 40 ml water) is added and the mixture is stirred at 32°C for 24 h while maintaining the pH of the solution at 7.5 with 38.7 ml of a 0.1M solution of NaOH in water. Immobilized Alcaligenes faecalis penicillin G acylase is removed by filtration to give a clear solution. HPLC assay of this solution indicated the presence of 288 mg (1.89 mmoles, 94.5%) of mandelic acid. After lyophilization, ¹H NMR assay of the thus obtained white solid indicated the presence of 416 mg (1.81 mmoles, 90.5%) of 7β-aminocephalosporanic acid-1β-oxide.

### Example 13

### Enzymatic hydrolysis of the D-α-hydroxyphenylacetyl side chain of the monosodium salt of 7β-(D-α-hydroxyphenylacetamido)-3'-[(1-sulfomethyltetrazol-5-yl)thio]desacetoxycephalosporanic acid

A solution of 559 mg (0.99 mmoles) of the monosodium salt of 7β-(D-α-hydroxyphenylacetamido)-3'-[(1-sulfomethyltetrazol-5-yl)thio]desacetoxycephalosporanic acid in 10 ml water is is brought to 32°C and the pH adjusted to 7.5 by the addition of a 0.1M solution of NaOH in water. Immobilized Alcaligenes faecalis penicillin G acylase (1.0 g, weighed after filtering and washing the beads with 20 ml water) is added and the mixture is stirred at 32°C for 1 h while maintaining the pH of the solution at 7.5 with 10.5 ml of a 0.1M solution of NaOH in water. Immobilized Alcaligenes faecalis penicillin G acylase is removed by filtration to give a clear solution. HPLC assay of this solution indicated the presence of 146 mg (0.96 mmoles, 97.0%) of mandelic acid.

### Example 14

### Enzymatic hydrolysis of the α-chlorophenylacetyl side chain of 6β-(α-chlorophenylactamido)penicillanic acid

A solution of 0.2062 g (0.41 mmoles; 16.6 mM) of the potassium salt of 6β-(α-chlorophenylactamido)penicillanic acid in 25 ml water is brought to 34°C and the pH is adjusted to 7.5 by addition of a 97.29 mM solution of KOH in water. Immobilized Alcaligenes faecalis penicillin G acylase (1.0 g, weighed after filtering and washing the beads with water) is added and the mixture is stirred at 34°C for 70 minutes while maintaining the pH of the solution at 7.5 with a 97.29 mM solution of KOH in water. After approximately 30 minutes (0.49 mmoles of KOH added to the reaction mixture) the rate of KOH consumption reaches a constant value, caused by the formation of HCl as a result of the chemical hydrolysis of α-chlorophenylacetic acid.

### Example 15

### Enzymatic hydrolysis fo the 2-thiopheneacetyl side chain of 6β-(2-thiopheneacetamido)penicillanic acid

A solution of 0.4089 g (1.01 mmoles; 40.2 mM) of the potassium salt of 6β-(2-thiopheneacetamido)penicillanic acid in 25 ml water is brought to 34°C and the pH is adjusted to 7.5 by addition of a 97.29 mM solution of KOH in water. Immobilized Alcaligenes faecalis penicillin G acylase (1.0 g, weighed after filtering and washing the beads with water) is added and the mixture is stirred at 34°C while maintaining the pH of the solution at 7.5 with a 97.29 mM solution of KOH in water until the KOH consumption stops. This point is reached after adding 1.01 mmoles of KOH.

### Example 16

### Enzymatic hydrolysis of the phenylacetic side chain of 3-(4-carbamoylquinuclidino-1-ylmethyl)-7β-phenylacetamido-3-cefem-4-carboxylate

A solution of 0.049 g (0.07 mmoles; 2.66 mM) of 3-(4-carbamoylquinuclidino-1-ylmethyl)-7β-phenylacetamido-3-cefem-4-carboxylate in 25 ml water is brought to 34°C and the pH is adjusted to 7.5 by addition of a 8.14 mM solution of KOH in water. Immobilized Alcaligenes faecalis penicillin G acylase (1.0 g, weighed after filtering and washing the beads with water) is added and the mixture is stirred at 34°C while maintaining the pH of the solution at 7.5 with a 8.14 mM solution of KOH in water until the KOH consumption stops. This point is reached after adding 0.074 mmoles of KOH.

## Claims

1. Use of Alcaligenes faecalis penicillin G acylase for the hydrolysis process of the 6- or 7-side chain of, respectively, the penam or cephem compounds of formula (I): 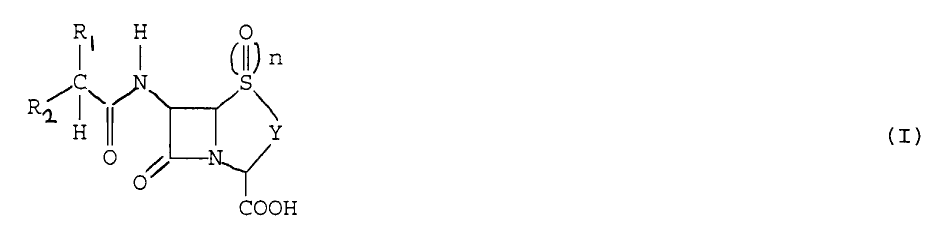 with
R₁ is hydrogen, hydroxy, amine, halogen or lower alkyl of 1-6 carbon atoms;
R₂ is an optionally substituted phenyl or 5- or 6-membered heterocyclic ring;
Y is 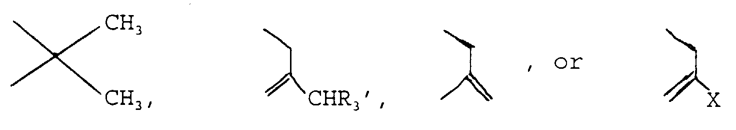 with
R₃' is lower alkylidene of 1-6 carbon atoms; and
X is hydrogen, halogen or lower alkoxy of 1-6 carbon atoms or optionally substituted methyl;
n is 1 or 2.

2. Use of Alcaligenes faecalis penicillin G acylase according to claim 1, characterized by the hydrolysis process of 6β-phenylacetamidopenicillanic acid 1,1-dioxide, 6β-phenylacetamidopenicillanic acid-1β-oxide, 7β-phenylacetamidodesacetoxycephalosporanic acid-1β-oxide, 7β-phenylacetamidodesacetoxycephalosporanic acid-1α-oxide, 7β-(α-bromophenylacetamido) desacetoxycephalosporanic acid-1β-oxide.

3. A process for the production of a penam or cephem compound according to formula (II): 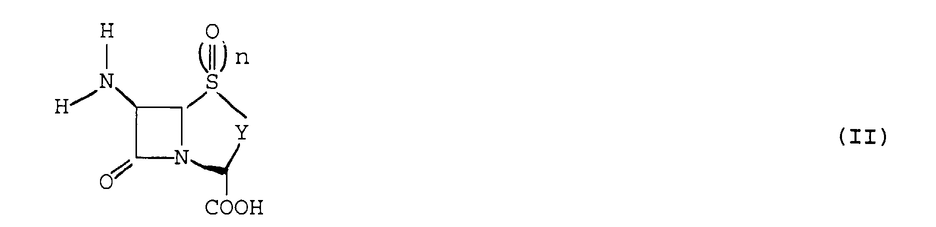 comprising the hydrolysis of the 6- or 7-side chain of, respectively, the penam or cephem compounds of formula (I): 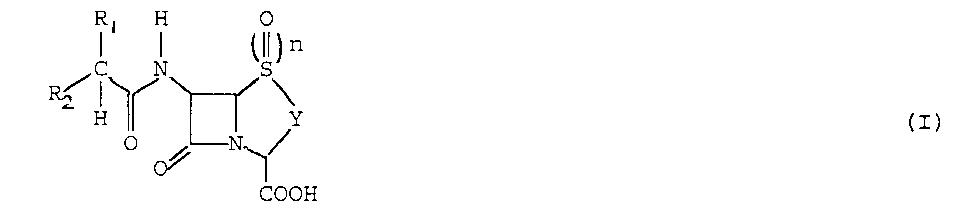 with R₁, R₂, Y, R₃', X and n as defined in claim 1,
wherein said hydrolysis is carried out by the penicillin G acylase of Alcaligenes faecalis.

## Patentansprüche

1. Verwendung von Alcaligenes faecalis Penicillin G Acylase für das Hydrolyseverfahren der 6- oder 7-Seitenkette der Penam- oder Cephemverbindungen der Formel (I) 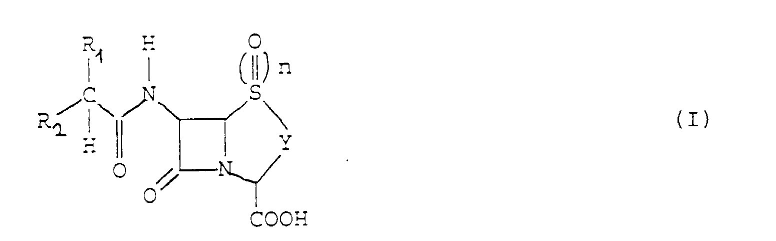 worin
R₁ Wasserstoff, Hydroxy, Amin, Halogen oder Niederalkyl mit 1 bis 6 Kohlenstoffatomen,
R₂ einen gegebenenfalls substituierten Phenylring oder einen 5- oder 6-gliedrigen heterocyclischen Ring,
Y einen der Reste 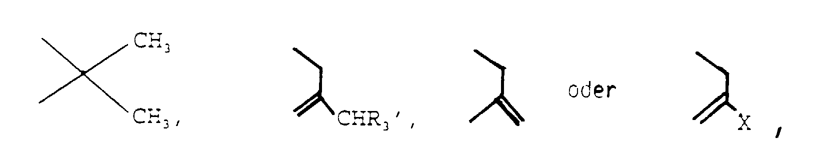 worin R₃' einen Niederalkylidenrest mit 1 bis 6 Kohlenstoffatomen und X Wasserstoff, Halogen oder Niederalkoxy mit 1 bis 6 Kohlenstoffatomen oder ein gegebenenfalls substituiertes Methyl darstellt, und
n die Zahl 1 oder 2
bedeuten.

2. Verwendung von Alcaligenes faecalis Penicillin G Acylase gemäß Anspruch 1, gekennzeichnet durch das Hydrolyseverfahren von 6β-Phenylacetamidopenicillansäure-1,1-dioxid, 6β-Phenylacetamidopenicillansäure-1β-oxid, 7β-Phenylacetamidodesacetoxycephalosporansäure-1β-oxid, 7β-Phenylacetamidodesacetoxycephalosporansäure-1α-oxid, 7β-(α-Bromphenylacetamido)-desacetoxycephalosporansäure-1β-oxid.

3. Verfahren zum Herstellen einer Penam- oder Cephemverbindung gemäß der Formel (II) 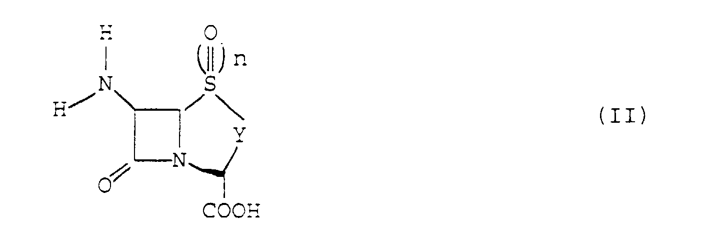 wobei die Hydrolyse der 6- oder 7-Seitenkette der Penam- oder Cephemverbindung der Formel (I) 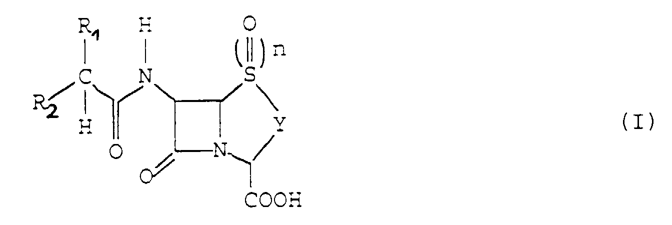 erfolgt und wobei R₁, R₂, Y, R₃', X und n wie im Anspruch 1 definiert sind sowie die Hydrolyse durch die Penicillin G Acylase von Alcaligenes faecalis durchgeführt wird.

## Revendications

1. Utilisation de pénicilline G acylase de Alcaligenes faecalis pour le processus d'hydrolyse de la chaîne latérale 6 ou 7 de, respectivement, les composés penam ou cephem de formule (I) : 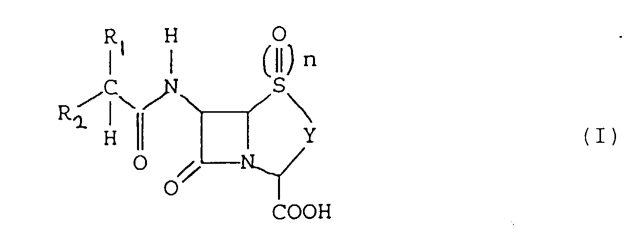 dans laquelle
R₁ est hydrogène, hydroxy, amine, halogène ou alkyle inférieur de 1-6 atomes de carbone;
R₂ est un phényle facultativement substitué ou un hétérocycle à 5 ou 6 membres;
Y est 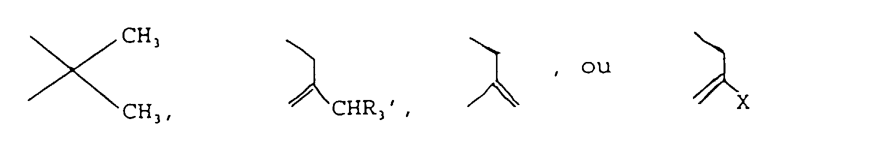 dans lesquels
R₃' est un alkylidène inférieur de 1-6 atomes de carbone, et
X est hydrogène, halogène ou alkoxy inférieur de 1-6 atomes de carbone ou un méthyle facultativement substitué;
n est 1 ou 2.

2. Utilisation de pénicilline G acylase de Alcaligenes faecalis suivant la revendication 1, caractérisée par le processus d'hydrolyse du 1,1-dioxyde de l'acide 6β-phénylacétamidopénicillanique, du 1β-oxyde de l'acide 6β-phénylacétamidopénicillanique, du 1β-oxyde de l'acide 7β-phénylacétamidodésacétoxycéphalosporanique, du 1α-oxyde de l'acide 7β-phénylacétamidodésacétoxycéphalosporanique, du 1β-oxyde de l'acide 7β-(α-bromophénylacétamido)désacétoxycéphalosporanique.

3. Procédé de production d'un composé de penam ou de cephem suivant la formule (II) : 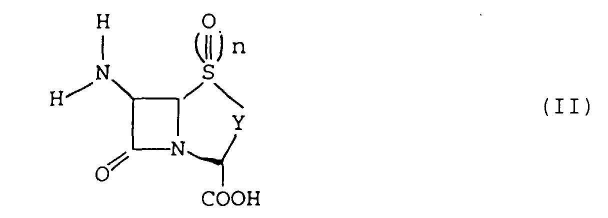 comprenant l'hydrolyse de la chaîne latérale 6 ou 7 de, respectivement, les composés penam ou cephem de formule (I) : 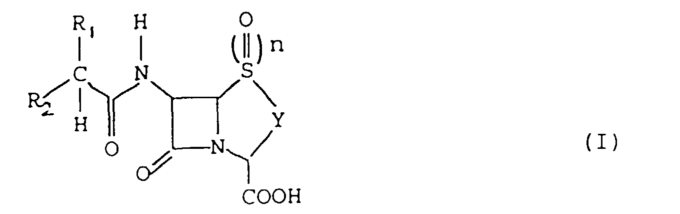 avec R₁, R₂, Y, R₃', X et n suivant la revendication 1, ladite hydrolyse étant réalisée par la pénicilline G acylase de Alcaligenes faecalis.
